# EUROPEAN PATENT SPECIFICATION

(11) **EP 3 409 248 B1**
(45) Date of publication and mention of the grant of the patent: **06.11.2019**
(21) Application number: 17174076.4
(22) Date of filing: 01.06.2017
(51) Int. Cl.: A61F 13/00, A61F 13/02

(54) **WOUND DRESSING**
WUNDAUFLAGE
PANSEMENT DE PLAIE

(43) Date of publication of application: 05.12.2018
(73) Proprietor: Absorbest AB, 590 39 Kisa (SE)
(72) Inventor: ROVANIEMI, Rolf, 590 46 Rimforsa (SE)
(74) Representative: WSL Patentanwälte Partnerschaft mbB

(56) References cited:
- EP-A1- 3 072 483
- WO-A2-2009/019229
- US-A1- 2010 204 667

## Description

The present invention relates to a wound dressing comprising an absorbent core with a top carrier layer and a bottom carrier layer carrying a superabsorbent substance dispersed between the top carrier layer and the bottom carrier layer, and a shell formed by a facing layer and a backing layer, wherein the absorbent core is located in the shell between the facing layer and the backing layer, wherein the facing layer and the backing layer each comprise a supporting structure, and wherein the supporting structure of the facing layer and of the backing layer each comprise an inner surface facing towards the absorbent core and an outer surface facing away from the absorbent core.

Wound dressings for covering wounds are known in various forms from the prior art. When applied to a wound, the facing layer of the wound dressing faces the wound. Wound fluids, such as wound exudate and blood, exuding from the wound flow through the facing layer into the absorbent core located within the shell formed by the facing layer and the backing layer. Wound fluids reaching the absorbent core are absorbed and fixed by the superabsorbent substance, such that the wound fluids can securely be removed together with the wound dressing.

The backing layer of the wound dressing might help to protect the absorbent core and to prevent the wound from being contaminated with foreign matter, germs, or the like contained in the proximity of the wound. Additionally, the backing layer is intended to protect the clothing of a patient wearing the wound dressing.

However, once a wound dressing is applied to a wound the wound dressing itself may cause discomfort to a patient. Since the facing layer is in contact with the wound, a movement of the patient might cause a relative movement between the wound and the wound dressing thereby stressing the wound. Stress to the wound might cause pain and discomfort to the patient. Furthermore, pressure applied to the wound dressing might cause an outflow of wound fluids from the wound dressing bearing the risk of a re-contamination of the wound.

When compared to the prior art it is an object of the present invention to provide an improved wound dressing, which is capable of reducing a wearer's discomfort and/or helps to prevent a re-contamination of the wound by wound fluids flowing out from the wound dressing and returning into the wound.

At least one of the above objects is solved by a wound dressing according to the preamble of claim 1, wherein at least one of the outer surface of the supporting structure of the facing layer and the outer surface of the supporting structure of the backing layer is laminated with a foam layer.

The foam layer laminated onto the supporting structure of the facing layer forms an outer surface of the facing layer, which in use of the dressing faces towards the wound. The foam layer is a barrier, which, compared to the supporting structure of the facing layer, is soft and capable to nestle up against the surface of the wound. The foam layer thus protects a wound and helps to reduce stress exerted to the wound caused by a movement of the wound dressing relative to the wound.

A foam layer laminated onto the outer surface of the supporting structure of the backing layer forms an outer surface of the backing layer, which in use of the dressing faces away from the wound. The foam layer, compared to the supporting structure of the backing layer, is soft and absorbs pressure exerting onto the wound dressing. The foam layer provides a wound healing friendly environment by balancing the moisture of the wound bed.

In the meaning of the present invention a foam layer laminated onto a supporting structure means that the surface of the foam layer and the surface of the supporting structure facing towards each other are joined together over a large area, i.e. over an area which is at least 1/3 of the overall surface of the foam layer. In particular, a laminate may be obtained by joining the surfaces of the foam layer and the supporting structure together by applying heat, pressure, or adhesives or a combination thereof.

In case both the outer surface of the supporting structure of the facing layer and the outer surface of the supporting structure of the backing layer are laminated with a foam layer, the wound dressing is turnable, such that the facing layer and the backing layer can be used to be laid onto a wound. A wound dressing having a facing layer and a backing layer comprising a foam layer laminated on each of the outer surface of the respective supporting structure of the facing layer and the backing layer, is multifariously useable.

In an embodiment of a wound dressing according to the present invention, the foam layer laminated onto the outer surface of the supporting structure of the facing layer and/or the foam layer laminated onto the outer surface of the supporting structure of the backing layer is a polyurethane foam, preferably a hydrophilic polyurethane foam.

In an embodiment the polyurethane foam comprises a fluid capacity after drain larger than 17 g/g determined by applying a test method based on EN 13726-1:2002-06, and/or a fluid capacity after compression larger than 9 g/g determined by applying a test method based on EN 13726-1:2002-06, and/or a tensile strength in the transversal and longitudinal direction larger than 50 kPa determined by applying a test method based on ISO 1798 knife-1:2008-04, and/or an elongation in the transversal and longitudinal direction of the laminated foam layer of 175 % determined by applying a test based on ISO 1798 knife 1:2008-04. The polyurethane foam may also comprise an average pore size in a range from 160 µm to 240 µm.

In any event, when applied to a patient's wound, wound fluids, such as wound exudate and blood, expelled from a wound covered by the wound dressing flow through the facing layer or the backing layer, when facing a wound, into the shell formed by the facing layer and the backing layer. The supporting structure supports the respective laminated foam layer and reinforces the facing and backing layers, irrespective of whether the respective foam layers of the wound dressing are saturated with wound fluids or not.

In an embodiment of a wound dressing according to the present invention, the supporting structure of the facing layer and/or the supporting structure of the backing layer is a hydrophilic or hydrophobic non-woven material, preferably the hydrophilic or hydrophobic non-woven material is one of a polypropylene (PP) spunbond, a combined non-woven fabric comprising a layer of a meltblown between two layers of a spunbond non-woven (SMS), a hydroentangled non-woven or a combined non-woven fabric comprising, in alternating order, three spunbond non-woven layers and two meltblown non-woven layers (SMSMS).

In an embodiment of a wound dressing according to the present invention, the supporting structure is a non-woven material, a perforated sheet, a perforated sheet laminated on a non-woven material, a fine net or screen, or a combination thereof. In an embodiment the non-woven material contains polyethylene (PE), polyethylene terephthalate (PET), polypropylene (PP), polyamide or polytetrafluoroethylene (PTFE). In an embodiment the perforated sheet contains polyethylene (PE), polyethylene terephthalate (PET), polypropylene (PP), poly-amide or polytetrafluoroethylene (PTFE). In an embodiment the perforated sheet laminated on a non-woven material contains polyethylene (PE), polyethylene terephthalate (PET), polypropylene (PP), polyamide or polytetrafluoroethylene (PTFE). In an embodiment the fine net or screen contains polyethylene (PE), polyethylene terephthalate (PET), polypropylene (PP), polyamide or polytetrafluoroethylene (PTFE).

In the meaning of the present invention a non-woven is a material made of at least one layer of undirected fibers that have been formed to a web and consolidated in a next step. In particular, consolidation of the non-woven material may be achieved by friction and/or cohesion and/or adhesion, for example by needling, felting, spunlacing, melting or heat embossing. When spunlacing is achieved by using water jets the non-woven is called a hydroentangled non-woven.

If compared to tissue paper a material will be considered a non-woven fabric in the sense of the present application once more than 50 % of the mass of its fiber components consist of fibers having a ratio of their length to their diameter of more than 300. Alternatively, the material will be considered a non-woven fabric in the sense of the present application if this condition is not fulfilled, but if more than 30 % of the mass of its fiber components consist of fibers having a ratio of their length to their diameter of more than 300 and its density is lower than 0.4 g / cm³. This is deemed to be in conformity with EN 29 092.

In an embodiment of a wound dressing according to the present invention, the supporting structure of the backing layer is made of a non-woven with a textile or a textile laminated backing sheet, preferably a textile backsheet (TBS) or a breathable textile backsheet (BTBS). A textile backsheet is a laminate of a non-woven and a plastic film, e.g. film made of polypropylene or polyethylene. A breathable textile backsheet (BTBS) is a laminate of a polypropylene non-woven and a microporous polyethylene film. The size of the micropores of the polyethylene film is chosen so to block water in its liquid form, but to allow transition of water vapor. By being water impermeable but water vapor permeable, the polyethylene film also functions as a sterile barrier blocking germs and the like.

In an embodiment of a wound dressing according to the present invention, the foam layer laminated onto the outer surface of the supporting structure of the facing layer and/or the foam layer laminated onto the outer surface of the supporting structure of the backing layer comprises a silicone coating on the outer surface of the respective foam layer laminated to the outer surface of the supporting structure of the facing layer or the outer surface of the supporting structure of the backing layer, respectively. Due to its non-absorbing capability, the silicone coating is less prone to be populated by bacteria. In particular, a silicone coating provides a non-sticking surface reducing the adhesion of the wound dressing to a wound surface thereby reducing pain implied to a patient, when the wound dressing is removed from the wound.

In an embodiment of a wound dressing according to the present invention the foam layer laminated onto the outer surface of the supporting structure of the facing layer and/or the foam layer laminated onto the outer surface of the supporting structure of the backing layer comprises a perforated silicone film or a mesh made of a silicone facing away from the absorbent core. A facing layer or a backing layer comprising a perforated silicone film comprises, in this order from the absorbent core in an outwards direction, the supporting structure laminated with a foam layer and additionally laminated with a perforated silicone film onto the foam layer, if applicable. Accordingly, a facing layer or a backing layer comprising a mesh made of a silicone comprises, in this order from the absorbent core in an outwards direction, the supporting structure laminated with a foam layer and additionally laminated with a mesh made of a silicone onto the foam layer, if applicable.

In an embodiment of a wound dressing according to the present invention the foam layer laminated onto the outer surface of the supporting structure of the facing layer and/or the foam layer laminated onto the outer surface of the supporting structure of the backing layer comprises a perforated silicone film or a mesh made of a silicone facing away from the absorbent core, wherein the perforated silicone layer or the mesh made of a silicone covers the respective surface of the facing layer or backing layer. In an embodiment of a wound dressing according to the present invention the perforated silicone layer or the mesh made of a silicone forms a ring-like structure, such that in a central part of the ring-like structure the respective facing layer or backing layer in the area of the absorbent core is exposed for accommodating an expansion of the wound dressing when the absorbent core during use of the wound dressing absorbs exudate from a wound. In a plan view onto the respective facing layer or backing layer the ring-like structure of the perforated silicone layer or the mesh made of a silicone surrounds the absorbent core.

In an embodiment of a wound dressing according to the present invention only the facing layer or the backing layer comprises a foam layer laminated onto the outer surface of the respective supporting structure, wherein the foam layer and the outer surface of the supporting structure not laminated with a foam layer comprise a perforated silicone film or a mesh made of a silicone facing away from the absorbent core. Hence, the wound dressing can be used either by laying the foam layer comprising the perforated silicone film or mesh onto a patient's wound or by laying the outer surface of the supporting structure comprising the perforated silicone film or mesh onto a patient's wound.

In any event, the perforated silicone film or the mesh of a silicone faces away from the absorbent core and forms the outer surface of the facing layer or backing layer, respectively. A perforated silicone film or a mesh made of a silicone might be used to adhere the wound dressing to a patient's skin, while the perforations of the perforated silicone film or the spacing of the mesh allows a flow of wound fluid through the respective perforated silicone film or mesh. A perforated silicone film or a mesh made of a silicone laminated onto the foam layer, if applicable, or the supporting structure of the backing layer, might be used to adhere the wound dressing to a bandage, or the like, for fixation of the wound dressing.

In an embodiment of a wound dressing according to the present invention the silicone coating and/or the perforated silicone film of the facing layer comprises a pressure sensitive adhesive for fixing the wound dressing to a patient's skin. The pressure sensitive adhesive can be particularly used to for a non-permanent fixation of the wound dressing, wherein a non-permanent fixation in the meaning of the present invention is a fixation which can be removed from a patient's skin without leaving a residual adhesive on the patient's skin.

In an embodiment of a wound dressing according to the present invention the foam layer laminated onto the supporting structure of the facing layer and/or the foam layer laminated onto the supporting structure of the backing layer comprises a bacteria adsorbing composition. A bacteria adsorbing substance, such as dialkylcarbamoyl chloride or dioctadecylcarbamoyl chloride, helps to prevent a wound covered by the wound dressing to be infected by bacteria.

In an embodiment of a wound dressing according to the present invention the supporting structure of the facing layer and/or the supporting structure of the backing layer comprises a bacteria adsorbing composition, such as for example dialkylcarbamoyl chloride or dioctadecylcarbamoyl chloride.

In an embodiment of a wound dressing according to the present invention the wound dressing comprises an adhesive for attaching the wound dressing to a patient's skin such that the facing layer faces the skin, wherein the adhesive partly covers the outer surface of the facing layer and/or forms a ring-like structure, such that in a central part of the ring-like structure the facing layer is exposed for accommodating an expansion of the wound dressing when the absorbent core during use of the wound dressing absorbs exudate from a wound.

In an embodiment of a wound dressing according to the present invention the wound dressing comprises an adhesive for attaching the wound dressing to a patient's skin such that the facing layer faces the skin, wherein the adhesive covers the outer surface of the backing layer and forms a ring-like structure extending beyond the area of the backing layer overlapping the absorbent core and facing towards the facing layer, such that in a central part of the ring-like structure the facing layer in the area of the absorbent core is exposed for accommodating an expansion of the wound dressing when the absorbent core during use of the wound dressing absorbs exudate from a wound. In a plan view onto the facing layer the ring-like structure of the adhesive surrounds the absorbent core.

In an embodiment of a wound dressing according to the present invention the wound dressing comprises an application aid in form of a pressure sensitive adhesive, wherein the pressure sensitive adhesive extends one the outer surface of the facing layer facing away from the absorbent core and wherein the total surface covered by the pressure sensitive adhesive is smaller than the surface of the bottom carrier layer of the absorbent core facing towards the facing layer.

In contrast to a wound dressing with a common permanent fixation on the wound, the application aid herein is defined by its characteristics of a non-permanent fixation enabling an easy repositioning and a regular replacement of the wound dressing. In order to fulfill this characteristics the application aid consists of a pressure sensitive glue attaching the wound dressing to the patient's skin, which has the essential property of a low adhesion when applied to the skin such that it peels off easily reducing pain induced on the patient.

In addition to a substantial decrease in pain for the patient, the regular change of the wound dressing made possible by the application aid has the consequence that a saturation of the absorbent core of the wound dressing, which is accompanied by a leakage of the absorbed wound fluid at the side portions of the wound dressing as well as by the risk of the accumulation of bacteria on the skin, is prevented. An improved healing of the wound may therefore be realized.

With a wound dressing according to the present invention fixation of the wound dressing is facilitated. Due to the pressure sensitive glue the wound dressing can reliably be brought onto the skin without getting out of place. However, if the wound dressing is misplaced, it can also easily be peeled off the skin due to its low adhesion leading to a substantial relief of pain and can be repositioned.

In one embodiment the pressure sensitive glue of the wound dressing has a 180° peel adhesion which is smaller than or equal to 0.68 N/mm, preferably smaller than or equal to 0.2 N/mm. preferably smaller than or equal to 0.06 N/mm and most preferably smaller than or equal to 0.04 N/mm. However, the 180° peel adhesion of the pressure sensitive glue exceeds 0.004 N/mm in order to provide at least a minimal adhesive contact between the wound dressing and the patient's skin. Consequently, the wound dressing with its application aid provides a low adhesion to the skin such that the removal and repositioning of the wound dressing does not cause a severe pain for the patient.

The 180° peel adhesion of the pressure sensitive glue given and discussed in this application is determined under the framework of the pressure sensitive tape council (PSTC) 101 test. A description of the test method is available online on the PSTC webpage, http://www.pstc.org/files/public/101.pdf, issued in October 2000 and last revised in May 2007.

In general, peel adhesion is defined as a measure of the strength of an adhesive bond between a tape and a test surface. Thus, the peel adhesion describes the force required to remove a pressure sensitive tape from a test surface at a controlled angle and at a standard rate. The dimension of the peel adhesion is given in force per width of the tape, resulting in the unit of measurement of Newton (N) per millimeter (mm).

In case of the 180° peel adhesion used for all values given in this application the test gives a measure of the adherence when peeled at 180° degree off a polished stainless steel test panel as test surface at a standard rate of 5 mm per second. The values of adhesion given herein were measured twenty minutes after the application of the pressure sensitive glue onto a test panel.

A quick adhesion of the wound dressing onto the patient's skin facilitates the fixation of the wound dressing onto the skin since the attachment occurs very fast. For example, a one-handed application of the wound dressing onto the wound can be realized very easily. Furthermore, the fast attachment to the skin minimizes the possibility that the wound dressing slips on the skin.

In an embodiment of a wound dressing according to the present invention, the facing layer and the backing layer are joined together by a circumferential hot melt thereby forming the shell enclosing the absorbent core. The shell enclosing the absorbent core prevents that part of the superabsorbent substance is accidentally released from the wound dressing, but maintained within the shell. Accordingly, the shell prevents an irritation of a wound, the wound dressing is applied to, which otherwise might be caused by an amount of lost superabsorbent substance entering the wound.

In an embodiment of a wound dressing according to the present invention, the absorbent core is fixed or freely moveable within the shell. For instance, the absorbent core can be fixed within the shell by an amount of a hot melt fixing the absorbent core to the supporting structure of the facing layer and/or to the supporting structure of the backing layer. In a particular embodiment the hot melt circumferentially applied to the supporting layer of the facing layer and to the supporting layer of the backing layer to form the shell extends onto the absorbent core thereby fixing the absorbent core to the shell.

The wound fluids entering the shell formed by the facing layer and the backing layer are absorbed by the superabsorbent substance dispersed between the top and bottom carrier layers of the absorbent core, such that the wound fluids can securely be removed together with the wound dressing.

Superabsorbent substances in the sense of the present application are materials being able to absorb and retain large volumes of water in aqueous solutions. Superabsorbent substances falling into this category are for example modified starch, polymerized polyvinyl alcohol (PVA) and polyethylene oxide (PEO) which are all hydrophilic and have a high affinity to water. When chemically or physically cross-linked, these polymers are water-swellable but not water-soluble. The aforementioned superabsorbent substances have been known for a long time.

In a particular embodiment of the present invention, the superabsorbent substance is a superabsorbent polymer (SAP), in particular in the form of (granular) particles or fibers. In an embodiment, such a SAP is made from polymerization of acrylic acids blended with sodium hydroxide in the presence of an initiated form poly-acrylic acid sodium salt (sometimes referred to a sodium polyacrylate).

In an embodiment, the carrier layers of the absorbent core in particular may comprise a material selected of a group consisting of tissue paper, a spunlaced polymer, a non-woven fabric, fluff/cellulose, regenerated cellulose as rayon, foam based on different chemistry as polyurethane, alginate, hydrocolloid, carboxymethyl cellulose (CMC) and its derivate and cotton. For manufacturing, the SAP is dispersed on the first layer, then the second layer is put on top and the two layers are consolidated providing a matrix carrying the SAP between the two layers. In a particular embodiment of a wound dressing according to the present invention, the top and bottom carrier layers of the absorbent core consist of 70 % by weight rayon and 30 % by weight polyester as a spun-laced material.

In an embodiment of a wound dressing according to the present invention the wound dressing comprises an amount of an active substance, preferably an amount of dialkylcarbamoyl chloride, dioctadecylcarbamoyl chloride, polyhexanide (PHMB), silver, antimicrobial peptide, copper, or zinc, integrated in one or more of the absorbent core, the facing layer, the backing layer and/or in parts thereof.

In an embodiment of a wound dressing according to the present invention the wound dressing comprises at least one sensor and a display, which is adapted to receive data determined by the sensor in form of an electrical signal and to display the received data.

The present invention also relates to the use of a wound dressing according to the present invention for the manufacturing of a bandage for the treatment of decubitus ulcers, venous skin ulcers, acute and chronic wounds, lymph edema, pressure ulcers, necrotic skin disease, low to high exuding wounds, wounds with a fibrin coating, postoperative wounds, open exuding wounds, or burns.

The present invention also relates to the use of a wound dressing according to the present invention to be applied to human or animal skin surfaces in the area of wounds so that the wound fluids exuding the wound are drawn into the shell, where the wound fluids are absorbed by the absorbent core, as a result of which a wound healing friendly environment is promoted, for the production of a bandage by means of which bioburdens are removed from the wound bed.

The present invention also relates to the use of a wound dressing according to the present invention in a method for treating a wound in a human or animal skin surfaces comprising the steps of providing a wound dressing according to the present invention, applying the wound dressing to a human or animal skin surface in the area of the wound to be treated, drawing wound fluids exuding from the wound to be treated into the shell by the means of the capillary effect of the laminated foam layer and/or by the means of the superabsorbent substance of the absorbent core, absorbing the wound fluids entering the shell by means of the absorbent core, as a result of which a wound healing friendly environment is promoted.

Further advantages, features and applications of the present disclosure will become apparent from the following description of embodiments and the corresponding figures attached, wherein in the figures identical elements are denoted by identical reference numerals. The foregoing as well as the following detailed description of the embodiments will be better understood when read in conjunction with the appended drawings. It should be understood that the embodiments depicted are not limited to the precise arrangements and instrumentalities shown.
- Figure 1: shows a schematic cross-sectional view of a wound dressing according to an embodiment of the present invention.
- Figure 2: shows a schematic cross-sectional view of a wound dressing according to another embodiment of the present invention.
- Figure 3: shows a schematic cross-sectional view of a wound dressing according to another embodiment of the present invention.
- Figure 4: shows a schematic cross-sectional view of a wound dressing according to another embodiment of the present invention.
- Figure 5: shows a schematic cross-sectional view of a wound dressing according to another embodiment of the present invention.

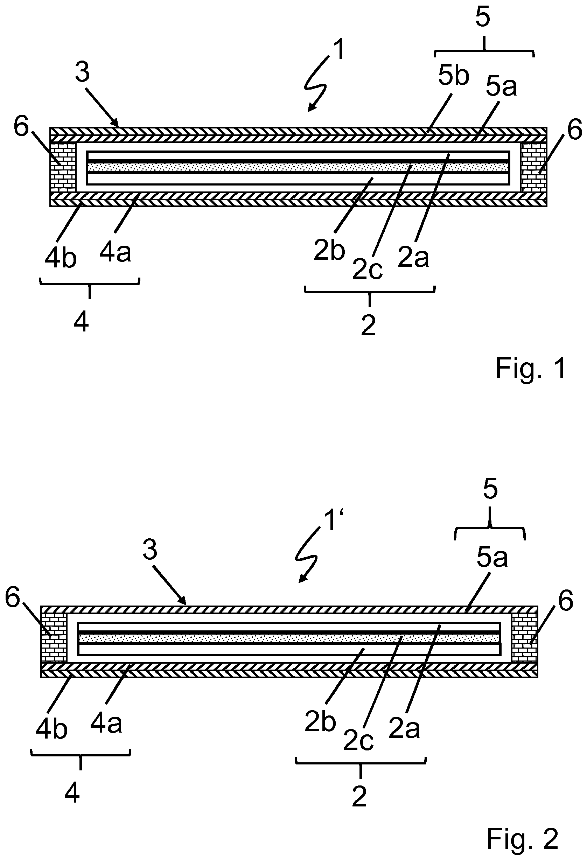

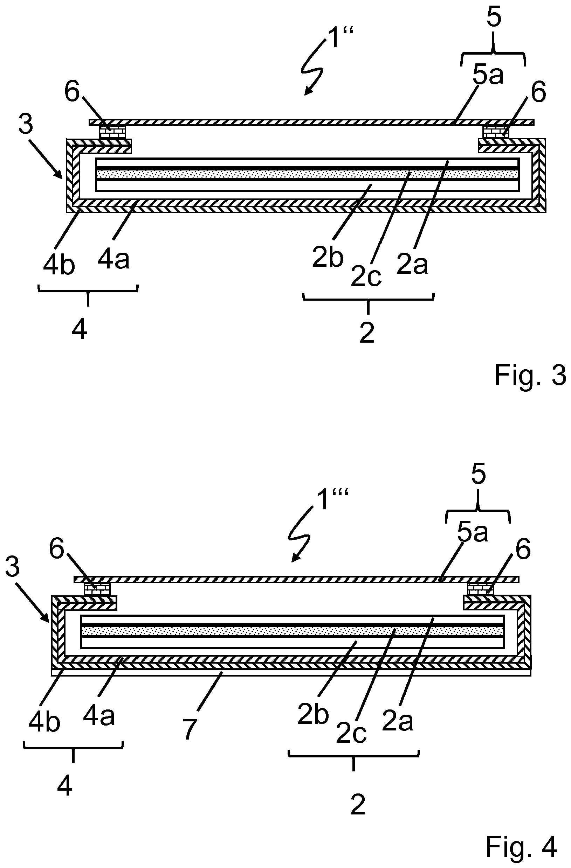

Each of figures 1 to 5 show a wound dressing 1, 1', 1", 1"', 1"" according to a different embodiment of the present invention. Figures 1 and 2 refer to wound dressings 1, 1' in a sandwich design, i.e. a design in which the different layers of the wound dressing are stacked onto each other. Figures 3 to 5 refer to wound dressings 1", 1"', 1"" in a tube design, i.e. a design in which part of the layers are folded onto each other. However, the features of respective wound dressings described herein are independent from the actual design of the wound dressing. The features of the wound dressings 1, 1' according to figures 1 and 2 could be implemented with a wound dressing in a tube design, too. Accordingly, the features of the wound dressings 1", 1"', 1"" according to figures 3 to 5 could be implemented with a wound dressing in a sandwich design, too.

Each of the wound dressings 1, 1', 1", 1"', 1"" comprises an absorbent core 2 with a top carrier layer 2a and a bottom carrier layer 2b carrying a superabsorbent substance 2c dispersed between the top carrier layer 2a and the bottom carrier layer 2b. Wound fluids, such as wound exudate and blood, reaching the absorbent core 2 are absorbed by the superabsorbent substance 2c.

The absorbent core 2 is encapsulated in a shell 3 formed by a facing layer 4 and a backing layer 5. The shell 3 functions as barrier and prevents that part of the superabsorbent substance 2c is accidentally released from the shell 3 and might enter a wound of a patient to whom the respective wound dressing 1, 1', 1", 1"', 1"" is applied to.

The facing layer 4 and the backing layer 5 each comprise a supporting structure 4a, 5a. The supporting structures 4a, 5a have an inner surface facing towards the absorbent core 2 and an outer surface facing away from the absorbent core 2. At least one of the outer surface of the supporting structure 4a of the facing layer 4 and the outer surface of the supporting structure 5a of the backing layer 5 is laminated with a foam layer 4b, 5b.

In the embodiment of a wound dressing 1 according to figure 1 both, the outer surface of the supporting structure 4a of the facing layer 4 and the outer surface of the supporting structure 5a of the backing layer 5 are laminated with a foam layer 4b, 5b. Accordingly, the wound dressing 1 may be applied to a wound of a patient's body by either covering the wound with the foam layer 4b laminated onto the outer surface of the supporting structure 4a of the facing layer 4 or by covering the wound with the foam layer 5b laminated onto the outer surface of the supporting structure 5b of the backing layer 5. In any event, wound fluids flow through the respective foam layer 4b, 5b and the respective supporting structure 4a, 5a into the absorbent core 2. The facing layer 4 and the backing layer 5 are joined together by a circumferential hot melt 6 gluing the inner surface of the supporting structure 4a of the facing layer 4 and the inner surface of the supporting structure 5a of the backing layer 5 to each other.

In the embodiment of a wound dressing 1' according to figure 2 only the outer surface of the supporting structure 4a of the facing layer 4 is laminated with a foam layer 4b. The outer surface of the supporting structure 5a of the backing layer 5 forms the outer surface of the wound dressing 1 facing away from the absorbent core 2 and the facing layer 4. Still, the facing layer 4 and the backing layer 5 are joined together by a circumferential hot melt 6 gluing the inner surface of the supporting structure 4a of the facing layer 4 and the inner surface of the supporting structure 5a of the backing layer 5 to each other.

In the particular embodiment shown in figure 2 the supporting structure 5a of the backing layer 5 is made from a breathable textile backsheet (BTBS), which is water impermeable, but water vapor permeable. In use of the wound dressing 1' the backing layer 5 helps to protect a patient's clothing and to establish a wound healing friendly environment underneath the wound dressing 1', i.e. on the side of the facing layer 4. The facing layer 4 and the backing layer 5 form the shell 3 encapsulating the absorbent core 2.

In the embodiments of a wound dressing 1", 1"', 1 "" according to figures 3 to 5 the facing layer 4 is folded in parallel to its edges such that the facing layer 4 also extends above the absorbent core 2, i.e. on the side of absorbent core 2 the backing layer 5 is located. The backing layer 5 partly overlaps with the sections of the facing layer 4 folded on top of the absorbent core 2. The overlapping sections of the backing layer 5 and the facing layer 4 are joined together by a hot melt 6 thereby sealing the shell 3. The absorbent core 2 is encapsulated by the shell 3.

Furthermore, in the embodiments of a wound dressing 1", 1"', 1 "" according to figures 3 to 5 the outer surface of the supporting structure 4a of the facing layer 4 is laminated with a foam layer 4b, while the outer surface of the supporting structure 5a of the backing layer 5 is not laminated with a foam layer 5b. However, the outer surface of the supporting structure 5a of the backing layer 5 might be laminated with a foam layer 5b, too, without deviating from the teaching of the present invention.

The embodiments shown in figures 3 to 5 differ in that they comprise additional features related to the facing layer 4. In the wound dressing 1" according to figure 3 the foam layer 4b laminated onto the outer surface of the supporting structure 4a of the facing layer 4 is the outermost layer facing away from the absorbent core 2. In use of the wound dressing 1", the foam layer 4b is intended to cover a patient's wound.

The facing layer 4 of the wound dressing 1'" according to figure 4 comprises a perforated silicone layer 7 laminated on the outer surface of the foam layer 4b of the facing layer 4. The perforated silicone layer 7 forms the outermost layer of the facing layer 4 facing away from the absorbent core 2. In use of the wound dressing 1"', the perforated silicone layer 7 is intended to cover a patient's wound.

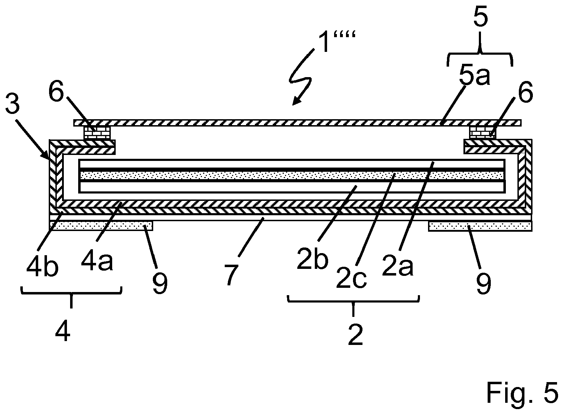

The facing layer 4 of the wound dressing 1"" according to figure 5 comprises a perforated silicone layer 7 laminated on the surface of the foam layer 4b of the facing layer 4, too. Additionally, the perforated silicone layer 7 comprises a pressure sensitive adhesive 9 for fixing the wound dressing 1"" to a patient's skin.

For purposes of original disclosure, it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations of features is only omitted in order to provide readability of the description.

While the disclosure has been described with respect to a limited number of embodiments, it will be understood that the disclosure of which is not limited to those embodiments. Other embodiments comprising various changes do not depart from the scope of the disclosure. In particular, the description of preferred embodiments shall not be understood to be limited to what is explicitly shown and described in the specification and drawings but shall encompasses the disclosure of the specification and drawings as a whole.

### List of reference numerals

- 1: Wound dressing
- 1', 1", 1"', 1"": Wound dressing
- 2: Absorbent core
- 2a: Top carrier layer of absorbent core
- 2b: Bottom carrier layer of absorbent core
- 2c: Superabsorbent substance dispersed between the top and bottom carrier layers
- 3: Shell
- 4: Facing layer
- 4a: Supporting structure of facing layer
- 4b: Foam layer of facing layer
- 5: Backing layer
- 5a: Supporting structure of backing layer
- 5b: Foam layer of backing layer
- 6: Hot melt
- 7: Perforated silicone film
- 8: Mesh made of a silicone
- 9: Pressure sensitive adhesive
- 10: Adhesive

## Claims

1. A wound dressing (1, 1', 1", 1"', 1"") comprising
an absorbent core (2) with
a top carrier layer (2a) and a bottom carrier layer (2b) carrying a superabsorbent substance (2c) dispersed between the top carrier layer (2a) and the bottom carrier layer (2b), and
a shell (3) formed by a facing layer (4) and a backing layer (5),
wherein the absorbent core (2) is located in the shell (3) between the facing layer (4) and the backing layer (5),
wherein the facing layer (4) and the backing layer (5) each comprise a supporting structure (4a, 5a), and
wherein the supporting structure (4a, 5a) of the facing layer (4) and of the backing layer (5) each comprise an inner surface facing towards the absorbent core (2) and an outer surface facing away from the absorbent core (2),
**characterized in that**
at least one of the outer surface of the supporting structure (4a) of the facing layer (4) and the outer surface of the supporting structure (5a) of the backing layer (5) is laminated with a foam layer (4b, 5b), wherein the surface of the foam layer (4b, 5b) and the surface of the supporting structure (4a, 5a) facing towards each other are joined together over an area which is at least 1/3 of the overall surface of the foam layer (4b, 5b).

2. The wound dressing (1, 1', 1", 1"', 1"") according to the previous claim, **characterized in that** the foam layer (4b) laminated onto the outer surface of the supporting structure (4a) of the facing layer (4) and/or the foam layer (5b) laminated onto the outer surface of the supporting structure (5a) of the backing layer (5) is layer is a polyurethane foam, preferably a hydrophilic polyurethane foam.

3. The wound dressing (1, 1', 1", 1"', 1"") according to the one of the previous claims, **characterized in that** supporting structure (4a) of the facing layer (4) and/or the supporting structure (5a) of the backing layer (5) is a hydrophilic or hydrophobic non-woven material, preferably the hydrophilic or hydrophobic non-woven material is one of a polypropylene (PP) spunbond, a combined non-woven fabric comprising a layer of a meltblown between two layers of a spunbond non-woven (SMS), a hydroentangled non-woven, or a combined non-woven fabric comprising, in alternating order, three spunbond non-woven layers and two meltblown non-woven layers (SMSMS).

4. The wound dressing (1, 1', 1", 1"', 1"") according to one of the previous claims, **characterized in that** the supporting structure (5a) of the backing layer (5) is a non-woven with a textile or a textile laminated backing sheet, preferably a textile backsheet (TBS) or a breathable textile laminated backsheet (BTBS).

5. The wound dressing (1, 1', 1", 1", 1"") according to one of the previous claims, **characterized in that** the foam layer (4b) laminated onto the outer surface of the supporting structure (4a) of the facing layer (4) and/or the foam layer (5b) laminated onto the outer surface of the supporting structure (5a) of the backing layer (5) comprises a silicone coating on the outer surface of the respective foam layer (4b, 5b) laminated to the outer surface of the supporting structure (4a) of the facing layer (4) and/or the outer surface of the supporting structure (5a) of the backing layer (5).

6. The wound dressing (1, 1', 1", 1'", 1"") according to one of the previous claims, **characterized in that** the foam layer (4b) laminated onto the outer surface of the supporting structure (4a) of the facing layer (4) and/or the foam layer (5b) laminated onto the outer surface of the supporting structure (5a) of the backing layer (5) comprises a perforated silicone film (8) or a mesh (8) made of a silicone facing away from the absorbent core (2).

7. The wound dressing (1, 1', 1", 1"', 1"") according to claims 5 or 6, **characterized in that** the silicone coating and/or the perforated silicone film (8) of the facing layer (4) comprises a pressure sensitive adhesive (9) for fixing the wound dressing (1, 1', 1", 1"', 1"") to a patient's skin.

8. The wound dressing (1, 1', 1", 1"', 1"") according to one the previous claims, **characterized in that** the wound dressing (1, 1', 1", 1"', 1"") comprises an amount of an active substance, preferably an amount of PHMB, silver, antimicrobial peptide, copper, or zinc, integrated in one or more of the absorbent core (2), the facing layer (4), the backing layer (5) and/or in parts thereof.

9. The wound dressing (1, 1', 1", 1"', 1"") according to one of the previous claims, **characterized in that** the foam layer (4b) laminated onto the supporting structure (4a) of the facing layer (4) and/or the foam layer (5b) laminated onto the supporting structure (5a) of the backing layer (5) comprises a bacteria adsorbing composition.

10. The wound dressing (1, 1', 1", 1"', 1"") according to the previous claim, **characterized in that** the facing layer (4) and the backing layer (5) are glued together by a circumferential hot melt (6) thereby forming the shell (3) enclosing the absorbent core (2).

11. The wound dressing (1, 1', 1", 1"', 1"") according to one of the previous claims, **characterized in that** the top and bottom carrier layers (2a, 2b) of the absorbent core consist of 70 % by weight rayon and 30 % by weight polyester as spun-laced material.

12. The wound dressing (1, 1', 1", 1'", 1"") according to one of the previous claims, **characterized in that** the wound dressing (1, 1', 1", 1 "', 1"") comprises an adhesive (10) for attaching the wound dressing (1, 1', 1", 1"', 1"") to a patient's skin such that the facing layer (4) faces the skin, wherein the adhesive partly covers the outer surface of the facing layer (4) and/or forms a ring-like structure, such that in a central part of the ring-like structure the facing layer (4) is exposed for accommodating an expansion of the wound dressing (1, 1', 1", 1"', 1"") when the absorbent core (2) during use of the wound dressing (1, 1', 1", 1'",1"") absorbs exudate from a wound.

13. The wound dressing (1, 1', 1", 1'", 1"") according to one of the previous claims, **characterized in that** the wound dressing (1, 1', 1", 1"', 1"") comprises an application aid in form of a pressure sensitive adhesive (9), wherein the pressure sensitive adhesive (9) extends one the outer surface of the facing layer (4) facing away from the absorbent core (2) and wherein the total surface covered by the pressure sensitive adhesive (9) is smaller than the surface of the bottom carrier layer (2b) of the absorbent core (2) facing towards the facing layer (4).

14. The wound dressing (1, 1', 1", 1'", 1"") according to one of the previous claims, **characterized in that** the wound dressing (1, 1', 1", 1"', 1"") comprises at least one sensor and a display, which is adapted to receive data determined by the sensor in form of an electrical signal and to display the received data.

## Patentansprüche

1. Wundauflage (1, 1', 1", 1"', 1"") mit
einem absorbierenden Kern (2) mit
einer oberen Trägerschicht (2a) und einer unteren Trägerschicht (2b), welche eine superabsorbierende Substanz (2c) tragen, welche zwischen der oberen Trägerschicht (2a) und der unteren Trägerschicht (2b) verteilt ist, und
einer Hülle (3), welche durch eine Deckschicht (4) und eine Trägerschicht (5) gebildet ist,
wobei der absorbierende Kern (2) in der Hülle (3) zwischen der Deckschicht (4) und der Trägerschicht (5) angeordnet ist,
wobei die Deckschicht (4) und die Trägerschicht (5) jeweils eine Trägerstruktur (4a, 5a) aufweisen, und
wobei die Trägerstruktur (4a, 5a) der Deckschicht (4) und der Trägerschicht (5) jeweils eine Innenfläche, welche in Richtung des absorbierenden Kerns (2) zeigt, und eine Außenfläche aufweisen, welche weg von dem absorbierenden Kern (2) zeigt,
**dadurch gekennzeichnet, dass**
mindestens eine der Außenfläche der Trägerstruktur (4a) der Deckschicht (4) und der Außenfläche der Trägerstruktur (5a) der Trägerschicht (5) mit einer Schaumschicht (4b, 5b) laminiert ist, wobei die Oberfläche der Schaumschicht (4b, 5b) und die Oberfläche der Trägerstruktur (4a, 5a), welche zueinander hin zeigen, über einen Bereich, der mindestens 1/3 der Gesamtfläche der Schaumschicht (4b, 5b) beträgt, miteinander verbunden sind.

2. Wundauflage (1, 1', 1", 1'", 1"") nach dem vorstehenden Anspruch, **dadurch gekennzeichnet, dass** die Schaumschicht (4b), welche auf die Außenfläche der Trägerstruktur (4a) der Deckschicht (4) laminiert ist und/oder die Schaumschicht (5b), welche auf die Außenfläche der Trägerstruktur (5a) der Trägerschicht (5) laminiert ist, ein Polyurethanschaum ist, vorzugsweise ein wasserabweisender Polyurethanschaum.

3. Wundauflage (1, 1', 1", 1"', 1"") nach einem der vorstehenden Ansprüche, **dadurch gekennzeichnet, dass** die Trägerstruktur (4a) der Deckschicht (4) und/oder die Trägerstruktur (5a) der Trägerschicht (5) ein hydrophiles oder ein hydrophobes Vliesmaterial ist, vorzugsweise ist das hydrophile oder hydrophobe Vliesmaterial eines aus einem Polypropylen-(PP)-Spinnvlies, einem kombinierten Vliesstoff mit einer Schicht aus Meltblown zwischen zwei Schichten von Spinnvliesen (SMS), einem wasservernadelten Vlies oder einem kombinierten Vliesstoff mit, in abwechselnder Reihenfolge, drei Spinnvliesschichten und zwei Meltblownvliesschichten (SMSMS).

4. Wundauflage (1, 1', 1", 1"', 1"") nach einem der vorstehenden Ansprüche, **dadurch gekennzeichnet, dass** die Trägerstruktur (5a) der Trägerschicht (5) ein Vlies mit einer textilen oder eine textile laminierte Rückseite, vorzugsweise eine textile Rückseite (TBS) oder eine atmungsaktive textile laminierte Rückseite (BTBS), ist.

5. Wundauflage (1, 1', 1", 1"', 1"") nach einem der vorstehenden Ansprüche, **dadurch gekennzeichnet, dass** die Schaumschicht (4b), welche auf die Außenfläche der Trägerstruktur (4a) der Deckschicht (4) laminiert ist und/oder die Schaumschicht (5b), welche auf die Außenfläche der Trägerstruktur (5a) der Tragschicht (5) laminiert ist, eine Silikonschicht auf der Außenfläche der entsprechenden Schaumschicht (4b, 5b), welche auf die Außenfläche der Trägerstruktur (4a) der Deckschicht (4) und/oder die Außenfläche der Trägerstruktur (5a) der Trägerschicht (5) laminiert ist, aufweist.

6. Wundauflage (1, 1', 1", 1"', 1"") nach einem der vorstehenden Ansprüche, **dadurch gekennzeichnet, dass** die Schaumschicht (4b), welche auf die Außenfläche der Trägerstruktur (4a) der Deckschicht (4) laminiert ist und/oder die Schaumschicht (5b), welche auf die Außenfläche der Trägerstruktur (5a) der Trägerschicht (5) laminiert ist, eine perforierte Silikonschicht (8) oder ein Netz (8) aus einem Silikon, welches weg von dem absorbierenden Kern (2) zeigt, aufweist.

7. Wundauflage (1, 1', 1", 1"', 1"") nach Anspruch 5 oder 6, **dadurch gekennzeichnet, dass** die Silikonschicht und/oder die perforierte Silikonschicht (8) der Deckschicht (4) einen selbstklebenden Klebstoff (9) zum Befestigen der Wundauflage (1, 1', 1", 1'", 1"") an der Haut eines Patienten aufweist.

8. Wundauflage (1, 1', 1", 1"', 1"") nach einem der vorstehenden Ansprüche, **dadurch gekennzeichnet, dass** die Wundauflage (1, 1', 1", 1"', 1"") eine Menge einer aktiven Substanz, vorzugsweise eine Menge an PHMB, Silber, antimikrobiellem Peptid, Kupfer oder Zink, aufweist, welche in einem oder mehreren aus dem absorbierenden Kern (2), der Deckschicht (4), der Trägerschicht (5) und/oder in Teilen davon integriert ist.

9. Wundauflage (1, 1', 1", 1"', 1"") nach einem der vorstehenden Ansprüche, **dadurch gekennzeichnet, dass** die Schaumschicht (4b), welche auf die Trägerstruktur (4a) der Deckschicht (4) laminiert ist und/oder die Schaumschicht (5b), welche auf die Trägerstruktur (5a) der Tragschicht (5) laminiert ist, eine Bakterien absorbierende Zusammensetzung aufweist.

10. Wundauflage (1, 1', 1", 1'", 1"") nach dem vorstehenden Anspruch, **dadurch gekennzeichnet, dass** die Deckschicht (4) und die Trägerschicht (5) durch einen umlaufenden Schmelzkleber (6) zusammengeklebt sind, um so die Hülle (3), welche den absorbierenden Kern (2) umgibt, zu bilden.

11. Wundauflage (1, 1', 1", 1"', 1"") nach einem der vorstehenden Ansprüche, **dadurch gekennzeichnet, dass** die oberen und unteren Trägerschichten (2a, 2b) des absorbierenden Kerns aus 70 Gew.-% Viskose und 30 Gew.-% Polyester als wasserstrahlverfestigtes Material bestehen.

12. Wundauflage (1, 1', 1", 1"', 1"") nach einem der vorstehenden Ansprüche, **dadurch gekennzeichnet, dass** die Wundauflage (1, 1', 1", 1"', 1"") einen Klebstoff (10) zum Befestigen der Wundauflage (1, 1', 1", 1'", 1"") an der Haut eines Patienten aufweist, sodass die Deckschicht (4) in Richtung der Haut zeigt, wobei der Klebstoff teilweise die Außenfläche der Deckschicht (4) bedeckt und/oder eine ringförmige Struktur bildet, sodass in einer Mitte der ringförmigen Struktur die Deckschicht (4) zur Aufnahme einer Ausdehnung der Wundauflage (1, 1', 1", 1"', 1"") freigelegt ist, wenn der absorbierende Kern (2) während der Verwendung der Wundauflage (1, 1', 1", 1"', 1"") Wundflüssigkeit von einer Wunde absorbiert.

13. Wundauflage (1, 1', 1", 1"', 1"") nach einem der vorstehenden Ansprüche, **dadurch gekennzeichnet, dass** die Wundauflage (1, 1', 1", 1'", 1"") eine Applikationshilfe in Form eines selbstklebenden Klebstoffs (9) aufweist, wobei der selbstklebende Klebstoff (9) sich auf der Außenfläche der Deckschicht (4) erstreckt, welche weg von dem absorbierenden Kern (2) zeigt, und wobei die Gesamtfläche, welche von dem selbstklebenden Klebstoff (9) bedeckt ist, kleiner ist als die Fläche der unteren Trägerschicht (2b) des absorbierenden Kerns (2), welche hin zu der Deckschicht (4) zeigt.

14. Wundauflage (1, 1', 1", 1"', 1"") nach einem der vorstehenden Ansprüche, **dadurch gekennzeichnet, dass** die Wundauflage (1, 1', 1", 1"', 1"") mindestens einen Sensor und eine Anzeige aufweist, welche dafür ausgelegt ist, Daten zu erhalten, welche durch den Sensor in Form eines elektrischen Signals bestimmt werden, und die erhaltenen Daten anzuzeigen.

## Revendications

1. Pansement pour plaies (1, 1', 1", 1"', 1"") comprenant
une âme absorbante (2) avec
une couche de support supérieure (2a) et une couche de support inférieure (2b) comportant une substance superabsorbante (2c) dispersée entre la couche de support supérieure (2a) et la couche de support inférieure (2b), et
une enveloppe (3) formée par une couche de surface (4) et une couche de renfort (5),
dans lequel l'âme absorbante (2) se trouve dans l'enveloppe (3) entre la couche de surface (4) et la couche de renfort (5),
dans lequel la couche de surface (4) et la couche de renfort (5) comportent chacune une structure de support (4a, 5a), et
dans lequel les structures de support (4a, 5a) de la couche de surface (4) et de la couche de renfort (5) comportent chacune une surface interne tournée vers l'âme absorbante (2) et une surface externe tournée dans la direction opposée à l'âme absorbante (2),
**caractérisé en ce que**
l'une au moins de la surface externe de la structure de support (4a) de la couche de surface (4) et de la surface externe de la structure de support (5a) de la couche de renfort (5) est laminée à l'aide d'une couche de mousse (4b, 5b), où la surface de la couche de mousse (4b, 5b) et la surface de la structure de support (4a, 5a), orientées l'une vers l'autre, sont assemblées sur une zone correspondant à au moins 1/3 de la surface globale de la couche de mousse (4b, 5b).

2. Pansement pour plaies (1, 1', 1", 1'", 1"") selon la revendication précédente, **caractérisé en ce que** la couche de mousse (4b) laminée sur la surface externe de la structure de support (4a) de la couche de surface (4) et/ou la couche de mousse (5b) laminée sur la surface externe de la structure de support (5a) de la couche de renfort (5) est une couche en mousse de polyuréthane, de préférence, une mousse de polyuréthane hydrophile.

3. Pansement pour plaies (1, 1', 1", 1"', 1"") selon l'une des revendications précédentes, **caractérisé en ce que** la structure de support (4a) de la couche de surface (4) et/ou la structure de support (5a) de la couche de renfort (5) est un matériau non tissé hydrophile ou hydrophobe, de préférence, le matériau non tissé hydrophile ou hydrophobe est un matériau en polypropylène (PP) filé-lié, un tissu non tissé combiné comprenant une couche d'un non-tissé obtenu par fusion-soufflage entre deux couches d'un non-tissé filé-lié (SMS), un non-tissé hydro-enchevêtré, ou un tissu non tissé combiné comprenant, alternativement, trois couches d'un non-tissé filé-lié et deux couches d'un non-tissé obtenu par fusion-soufflage (SMSMS).

4. Pansement pour plaies (1, 1', 1", 1"', 1"") selon l'une des revendications précédentes, **caractérisé en ce que** la structure de support (5a) de la couche de renfort (5) est un non-tissé avec une feuille de support textile ou laminée textile, de préférence un voile sensation textile «Textile Backsheet (TBS)» ou une Backsheet laminée textile respirante (BTBS).

5. Pansement pour plaies (1, 1', 1", 1"', 1"") selon l'une des revendications précédentes, **caractérisé en ce que** la couche de mousse (4b) laminée sur la surface externe de la structure de support (4a) de la couche de surface (4) et/ou la couche de mousse (5b) laminée sur la surface externe de la structure de support (5a) de la couche de renfort (5) comprend/comprennent un revêtement de silicone sur la surface externe de la couche de mousse respective (4b, 5b) laminée sur la surface externe de la structure de support (4a) de la couche de surface (4) et/ou la surface externe de la structure de support (5a) de la couche de renfort (5).

6. Pansement pour plaies (1, 1', 1", 1"', 1"") selon l'une des revendications précédentes, **caractérisé en ce que** la couche de mousse (4b) laminée sur la surface externe de la structure de support (4a) de la couche de surface (4) et/ou la couche de mousse (5b) laminée sur la surface externe de la structure de support (5a) de la couche de renfort (5) comprend/comprennent un film de silicone perforé (8) ou une maille (8) en silicone tourné(e) dans la direction opposée à l'âme absorbante (2).

7. Pansement pour plaies (1, 1', 1", 1"', 1"") selon la revendication 5 ou 6, **caractérisé en ce que** le revêtement de silicone et/ou le film de silicone perforé (8) de la couche de surface (4) comprend/comprennent un adhésif autocollant (9) pour fixer le pansement (1, 1', 1", 1"', 1"") sur la peau d'un patient.

8. Pansement pour plaies (1, 1', 1", 1"', 1"") selon l'une des revendications précédentes, **caractérisé en ce que** ledit pansement (1, 1', 1", 1'", 1"") comprend une quantité d'une substance active, de préférence, une quantité de PHMB, d'argent, de peptide antimicrobien, de cuivre ou de zinc, intégrée dans une ou plusieurs parmi l'âme absorbante (2), la couche de surface (4), la couche de renfort (5) et/ou dans des parties de celles-ci.

9. Pansement pour plaies (1, 1', 1", 1"', 1"") selon l'une des revendications précédentes, **caractérisé en ce que** la couche de mousse (4b) laminée sur la structure de support (4a) de la couche de surface (4) et/ou la couche de mousse (5b) laminée sur la structure de support (5a) de la couche de renfort (5) comprend/comprennent une composition absorbant les bactéries.

10. Pansement pour plaies (1, 1', 1", 1'", 1"") selon la revendication précédente, **caractérisé en ce que** la couche de surface (4) et la couche de renfort (5) sont collées ensemble à l'aide d'une masse fondue circonférentielle (6) formant ainsi l'enveloppe (3) entourant l'âme absorbante (2).

11. Pansement pour plaies (1, 1', 1", 1"', 1"") selon l'une des revendications précédentes, **caractérisé en ce que** les couches de support supérieure et inférieure (2a, 2b) de l'âme absorbante sont constituées de 70% en poids de rayonne et 30% en poids de polyester sous forme de matériau filé entrelacé.

12. Pansement pour plaies (1, 1', 1", 1"', 1"") selon l'une des revendications précédentes, **caractérisé en ce que** ledit pansement (1, 1', 1", 1"', 1"") comprend un adhésif (10) pour fixer le pansement (1, 1', 1", 1"', 1"") sur la peau d'un patient de sorte que la couche de surface (4) se trouve face à la peau, dans lequel l'adhésif recouvre partiellement la surface externe de la couche de surface (4) et/ou forme une structure annulaire, de sorte que dans une partie centrale de la structure annulaire, la couche de surface (4) est exposée pour permettre un élargissement du pansement (1, 1', 1", 1'", 1"") lorsque l'âme absorbante (2) absorbe lors de l'utilisation du pansement (1, 1', 1", 1"', 1"") l'exsudat provenant d'une plaie.

13. Pansement pour plaies (1, 1', 1", 1"', 1"") selon l'une des revendications précédentes, **caractérisé en ce que** ledit pansement (1, 1', 1", 1'", 1'''') comprend un dispositif d'aide à l'application sous forme d'un adhésif autocollant (9), dans lequel ledit adhésif autocollant (9) s'étend sur la surface externe de la couche de surface (4) tournée dans la direction opposée à l'âme absorbante (2) et dans lequel la surface totale recouverte par l'adhésif autocollant (9) est plus petite que la surface de la couche de support inférieure (2b) de l'âme absorbante (2) tournée vers la couche de surface (4).

14. Pansement pour plaies (1, 1', 1", 1"', 1"") selon l'une des revendications précédentes, **caractérisé en ce que** ledit pansement (1, 1', 1", 1"', 1"") comprend au moins un capteur et un afficheur, qui est adapté pour recevoir les données fournies par le capteur sous forme de signal électrique et pour afficher les données reçues.
